# EUROPEAN PATENT APPLICATION

(11) **EP 2 251 022 A1**
(43) Date of publication of application: **17.11.2010**
(21) Application number: 09159929.0
(22) Date of filing: 11.05.2009
(51) Int. Cl.: A61K 35/76, A61P 37/04

(54) **Non-replicating micro-organisms and their immune boosting effect**

(71) Applicant: Nestec S.A., 1800 Vevey (CH)
(72) Inventor: Prioult, Guénolée, 1005, LAUSANNE (CH); Mercenier, Annick, 1030, BUSSIGNY (CH); Nutten, Sophie, 1005, LAUSANNE (CH)
(74) Representative: Marquardt, Ulf

(57) **Abstract**

The present invention generally relates to the field of probiotic bacteria. In particular, the present invention concerns non-replicating probiotics, such as the genus *Lactobacillus, Bifidobacterium* or combinations thereof, for example *Lactobacillus paracasei, Lactobacillus rhamnosus, Bifidobacterium longum, Bifidobacterium lactis, Bifidobacterium breve* or combinations thereof, and applications of these bacteria. One embodiment of the present invention relates to non-replicating probiotics and their use to prepare a composition to treat or prevent disorders related to a compromised immune defence. A method to increase the effectiveness of probiotics to treat or prevent immune disorders is described.

## Description

The present invention generally relates to the field of micro-organisms, in particular food grade bacteria. One embodiment of the present invention concerns non-replicating probiotics belonging to genera such as *Lactobacillus, Bifidobacterium* or combinations thereof, for example the species *Lactobacillus paracasei, Lactobacillus rhamnosus, Bifidobacterium longum, Bifidobacterium lactis, Bifidobacterium breve* or combinations thereof, and applications of these bacteria. One embodiment of the present invention relates to non-replicating probiotics and their use to prepare a composition to treat or prevent disorders that are related to a compromised immune defence.

Probiotics may be defined as "Live microorganisms which when administered in adequate amounts confer a health benefit on the host" [FAO/WHO (2001) Health and Nutritional Properties of Probiotics in Food including Powder Milk with Live Lactic Acid Bacteria. Report of a Joint FAO/WHO Expert Consultation on Evaluation of Health and Nutritional Properties of Probiotics in Food Including Powder Milk with Live Lactic Acid Bacteria]. Therefore, the vast majority of published literature deals with living probiotics. However, several studies investigated the health benefits delivered by non-replicating bacteria but came to the conclusion that heat-inactivation of probiotics generally leads to a loss of their purported health benefit (Rachmilewitz, D., K. et al, 2004, Gastroenterology 126:520-528; Castagliuolo, I., et al., 2005, FEMS Immunol.Med.Microbiol. 43:197-204; Gill, H. S. and K. J. Rutherfurd. 2001, Br.J.Nutr. 86:285-289; Kaila, M., et al.,. 1995, Arch.Dis.Child 72:51-53; Wagner, R. D., et al., 2000, J.Food Prot. 63:638-644).

Some studies, however, showed that killed probiotics may retain some health effects. This may depend for example on the method used to inactivate them (Rachmilewitz, D., K. et al, 2004, Gastroenterology 126:520-528 ; Gill, H. S. and K. J. Rutherfurd. 2001, Br.J.Nutr. 86:285-289). The technologies used in the literature to kill probiotic strains are mostly heat-treatment, γ-irradiation, UV-treatment or chemical agents (formalin, paraformaldehyde).

Usually, in the food industry today, probiotics are added to food products that are either stored for longer times in a cold chain and/or are treated to reduce bacterial loads. However, reducing bacterial loads in a product, long storage times as well as any kind of processing, such as spray drying, etc., reduces the amount of viable probiotics in a product. Reducing the amount of viable probiotics in a product is, however, usually equated in the art with a reduction or even a loss of the purported health effect of the probiotics, as detailed above.

While findings that some preparations comprising specific killed probiotics may retain some health effects may be encouraging, live probiotics are generally considered as having better or at least the same effects as inactivated probiotics.

It would, however be desirable to have available microbial cell preparations which have improved or new health benefits after inactivation.

A compromised immune defence may have many negative effects on a subject's health and well-being. It may for example result in a greater risk for infections and/or in an increased severity of infections. It may also promote or reinforce immune deficiency related disorders, or lead to allergy.

Strengthening the immune defence is therefore important for all subjects at all age groups to protect the body. In particular, this is important for those subjects whose immune system is compromised or transiently depressed such as the neonates, the elderly, the subject submitted to high stress conditions, the patients taking immunosuppressive drugs, patients under radiotherapy or chemotherapy, or subjects developing allergic diseases.

Natural defences against infections and immune deficiency related diseases imply, among others, that the host is able to mount efficient and rapid innate immune defences that include activation of macrophages and natural killer cells for example. In addition efficient immune defences also imply that the host is able to downregulate an overreaction of the immune system such as that occurring in allergy.

The killing activity of macrophages in response to phagocytosis of pathogens is usually accompanied by a transient boost in pro-inflammatory cytokines such as TNF-α, IL-6, IL-1, and IL-12 (Shoda, L., et al., 2000, Infection and Immunity 68:5139-5145). IL-12 produced by antigen presenting cells including macrophages activates natural killer cells to produce IFN-γ and promotes the development of acquired immune responses through the differentiation of IFN-γ-producing T helper cells. In addition, TNF-α and IFN-γ acting in an autocrine loop stimulate the killing activity of phagocytic cells (Soehnlein, O., et al., 2008, Journal of Clinical Investigation 118:3491-3502). By contrast, IL-10 produced by many immune cell types inhibits the production of pro-inflammatory cytokines produced by macrophages and dendritic cells like IL-1, IL-6, IL-12 and TNF-α (Mosser, D., and Zhang, X., 2008, Immunological Reviews 226:205-218). Specific live probiotic strains are known to stimulate pro-inflammatory cytokines *in vitro* such as IL-12 and TNF-α which is linked to an enhanced phagocytosis activity of rat peritoneal macrophages (Ishida-Fujii, K., 2007, Biosc. Biotechnol. Biochem 71:866-873).

In humans (healthy adults and elderly), consumption of live probiotics or food products containing probiotics has been reported to enhance the phagocytic activity of macrophages and/or the killing activity of natural killer cells, suggesting a immune boost effect of some live probiotic strains (Sheih, Y-H., et al., 2001, J Ameriacn College Nutrition 20:149-156; Chiang, B., et al., 2000, European J Clinical Nutrition 54:849-855; Parra, M., et al., 2004, J Physiol Biochem 60:85-92; Nagao, F., et a., 2000, Biosc Biotechnol Biochem 64:2706-2708).

However, there remains a need in the art for microbial cell preparations comprising probiotics with a beneficial effect on the immune system of a host, wherein the cell preparations exert their beneficial effects only or particularly well if they were treated under conditions that do not allow microbial cells to replicate and/or to remain viable.

It was consequently an object of the present invention to further improve the state of the art and to provide a microbial cell preparation that exhibits novel or improved beneficial effects on the immune system of the host under conditions, where microbial cells were treated in a way that does not allow them to replicate and/or to remain viable.

This object was solved by the subject matter of the independent claims. The dependent claims further develop the present invention.

The prior art generally teaches that heat treatment of probiotics leads to a partial or complete loss of their health beneficial properties. Only in exceptional cases some health benefits tested were maintained (Verdu et al., 2004, Gastroenterology, 127, p.826 ff., Rousseaux, 2007, Nature Medicine, 13, p.35ff; Kamiya et al., 2006, Gut, 55, 191 ff.).

The present inventors were now surprised to see that the ability of probiotic strains to stimulate for example the production of pro-inflammatory cytokines by human cells can be enhanced after heat treatment. This effect has been observed for several lactobacilli and bifidobacteria.

Non-replicating probiotic micro-organisms have the advantage that they are far easier to handle than their live counterparts. Additionally, they are far more storage stable and need less stringent packaging conditions.

Non-replicating probiotic micro-organisms would allow developing a large variety of functional foods which by their nature do not allow the addition of live probiotics without additional measures to protect them. This plays a role for example in the provision of cereal bars, fruit juices, UHT-drinks, shelf stable drinks, etc.

Further, for example in immuno-compromised customers, the use of live probiotics might be limited due to a potential risk to develop bacteremia. Here the inventors present a method to generate non viable bacteria with an *in vitro* immune boosting profile regardless of their initial immune profiles. Bacteria with no immune boosting profile when they are alive may be provided with an immune boosting profile; and bacteria with an immune boosting profile when they are alive may be provided with an enhanced immune boosting profile.

Additionally, the provision of non-replicating probiotic micro-organisms allows the hot reconstitution, e.g., of powdered nutritional compositions.

It is hence now possible to generate non replicating probiotic micro-organisms that enhance the immune defence more efficiently than their live counterparts do.

While the prior art generally teaches that bacteria rendered non-replicating by heat-treatment are usually less efficient than live cells in terms of exerting their probiotic properties, the present inventors were able to demonstrate that heat-treated probiotics are superior in stimulating the immune system compared to their live counterparts.

The present invention relates hence to a composition comprising probiotics that are rendered non-replicating by a heat treatment of at least about 70°C for at least about 3 minutes.

It also relates to a composition comprising probiotics that are rendered non-replicating by a heat treatment of at least about 70°C for at least about 3 minutes for treating or preventing disorders related to a compromised immune defence.

The present invention concerns the use of probiotics that are rendered non-replicating by a heat treatment of at least about 70°C for at least about 3 minutes for the preparation of a composition to treat or prevent disorders related to a compromised immune defence.

Consequently, the non-replicating probiotics of the present invention may be used for the preparation of a composition to boost the immune defence.

"Non-replicating" means that no viable cells and/or colony forming units can be detected by classical plating methods. Such classical plating methods are summarized in the microbiology book: James Monroe Jay, Martin J. Loessner, David A. Golden. 2005. Modern food microbiology. 7th edition, Springer Science, New York, N.Y. 790 p. Typically, the absence of viable cells can be shown as follows: no visible colony on agar plates or no increasing turbidity in liquid growth medium after inoculation with different concentrations of bacterial preparations ('non replicating' samples) and incubation under appropriate conditions (aerobic and/or anaerobic atmosphere for at least 24h).

For example, bifidobacteria such as *Bifidobacterium longum, Bifidobacterium lactis* and *Bifidobacterium breve* or lactobacilli, such as *Lactobacillus paracasei* or *Lactobacillus rhamnosus,* may be rendered non-replicating by heat treatment, in particular low temperature/long time heat treatment.

At least 95 weight %, preferably at least 97.5 weight %, even more preferred at least 99 weight % of the biomass of probiotics are non-replicating, and most preferred all probiotics are non-replicating.

The probiotics are rendered non-replicating by a heat treatment. This heat treatment may be carried out in the temperature range of about 70-150 °C for about 3 minutes - 2 hours, preferably in the range of 80-140°C from 5 minutes - 40 minutes.

As it is clear for those of skill in the art, the longer the heat treatment and/or the higher the temperature the more bacterial cells or bacterial compounds will be damaged and/or released.

The probiotic may be selected from the group consisting of the genera lactobacilli, bifidobacteria or combinations thereof, such as the species *Lactobacillus paracasei, Lactobacillus rhamnosus, Bifidobacterium longum, Bifidobacterium lactis, Bifidobacterium breve* or combinations thereof, for example the strains *Lactobacillus paracasei* NCC2461, *Lactobacillus rhamnosus* NCC4007, *Bifidobacterium longum* NCC3001, *Bifidobacterium lactis* NCC2818, *Bifidobacterium breve* strain A or combinations thereof.

*Bifidobacterium longum* NCC3001 was deposited under the Budapest treaty as ATCC BAA-999.

*Bifidobacterium lactis* NCC2818 was deposited under the Budapest treaty as (CNCM I-3446).

*Lactobacillus rhamnosus* NCC4007 was deposited under the Budapest treaty as (CGMCC 1.3724).

*Lactobacillus paracasei* NCC2461 was deposited under the Budapest treaty as (CNCM I-2116).

The immune boosting effects of non-replicating probiotics were confirmed by *in vitro* immunoprofiling. The *in vitro* model used uses cytokine profiling from human Peripheral Blood Mononuclear Cells (PBMCs) and is well accepted in the art as standard model for tests of immunomodulating compounds (Schultz et al., 2003, Journal of Dairy Research 70, 165-173;Taylor et al., 2006, Clinical and Experimental Allergy, 36, 1227-1235; Kekkonen et al., 2008, World Journal of Gastroenterology, 14, 1192-1203)

The *in vitro* PBMC assay has been used by several authors/research teams for example to classify probiotics according to their immune profile, i.e. their anti- or pro-inflammatory characteristics (Kekkonen et al., 2008, World Journal of Gastroenterology, 14, 1192-1203). For example, this assay has been shown to allow prediction of an anti-inflammatory effect of probiotic candidates in mouse models of intestinal colitis (Foligne, B., et al., 2007, World J.Gastroenterol. 13:236-243). Moreover, this assay is regularly used as read-out in clinical trials and was shown to lead to results coherent with the clinical outcomes (Schultz et al., 2003, Journal of Dairy Research 70, 165-173; Taylor et al., 2006, Clinical and Experimental Allergy, 36, 1227-1235).

Allergic diseases have steadily increased over the past decades and they are currently considered as epidemics by WHO. In a general way, allergy is considered to result from an imbalance between the Th1 and Th2 responses of the immune system leading to a strong bias towards the production of Th2 mediators. Therefore, allergy can be mitigated, down-regulated or prevented by restoring an appropriate balance between the Th1 and Th2 arms of the immune system. This implies the necessity to reduce the Th2 responses or to enhance, at least transiently, the Th1 responses. The latter would be characteristic of an immune boost response, often accompanied by for example higher levels of IFNγ, TNF-α and IL-12. (Kekkonen et al., 2008, World Journal of Gastroenterology, 14, 1192-1203; Viljanen M. et al., 2005, Allergy, 60, 494-500)

The present invention allows it to treat or prevent disorders that are related to a compromised immune defence.

Consequently, the disorders linked to a compromised immune defence that can be treated or prevented by the composition prepared by the use of the present invention are not particularly limited.

For example, they may be selected from the group consisting of infections, in particular bacterial, viral, fungal and/or parasite infections; phagocyte deficiencies; low to severe immunodepression levels such as those induced by stress or immunodepressive drugs, chemotherapy or radiotherapy; natural states of less immunocompetent immune systems such as those of the neonates or elderly; allergies; and combinations thereof.

The composition described in the present invention allows it also to enhance a subject's response to vaccines, in particular to oral vaccines.

Likewise, the kind of composition that is prepared by the use of the present invention is not particularly limited. For example, it may be a pharmaceutical composition, a nutraceutical, a food additive, a pet food, a food product or a drink.

The composition of the present invention may be any kind of composition. The composition may be to be administered orally, enterally, parenterally (subcutaneously or intramuscularly), topically or ocularly, for example.

For example it may be a composition selected from the group consisting of food compositions, food products including pet foods, drinks, nutritional formulas, feeding formulas, nutraceuticals, food additives, pharmaceutical compositions, cosmetical compositions, and medicaments.

A food additive or a medicament may be in the form of tablets, capsules, pastilles or a liquid for example. These compositions may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. They may also contain conventional pharmaceutical additives and adjuvants, excipients and diluents, including, but not limited to, water, gelatine of any origin, vegetable gums, ligninsulfonate, talc, sugars, starch, arabic gum, vegetable oils, polyalkylene glycols, flavouring agents, preservatives, stabilizers, emulsifying agents, buffers, lubricants, colorants, wetting agents, fillers, and the like.

Further, the compositions may contain an organic or inorganic carrier material suitable for oral, sublingual, topical, ocular, enteral or parenteral (e.g. subcutaneous, intramuscular) administration as well as vitamins, minerals trace elements and other micronutrients in accordance with the recommendations of Government bodies such as the USRDA.

The composition of the present invention may further comprise other agents, depending on the intended use of the composition. For example a pain or fever relieving agent may be used to help minimize the uncomfortable feeling caused by the disorder related to a weakened immune defence.

A stabilizing agent may be added to stabilize the composition and its constituents.

A flavouring agent and/or a colouring agent may be added to adjust flavours and to give the composition a colour that is easy to identify and/or that is perceived as pleasant.

Prebiotics may be added. Prebiotics may support the growth of a probiotic before it is rendered non-replicating or, in case of ingestion, stimulate the growth of beneficial micro-organisms in the intestines. Prebiotics may also act synergistically with viable probiotic bacteria that are present in the composition and/or that may be added.

"Prebiotic" means non-digestible food substances that promote the growth of health beneficial micro-organisms and/or probiotics in the intestines. They are not broken down in the stomach and/or upper intestine or absorbed in the GI tract of the person ingesting them, but they are fermented by the gastrointestinal microbiota and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.

The prebiotics that may be used in accordance with the present invention are not particularly limited and include all food substances that promote the growth of probiotics and/or health beneficial bacteria in the intestines. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (GOS), isomalto-oligosaccharides (IMO), xylo-oligosaccharides (XOS), arabino-xylo oligosaccharides (AXOS), mannan oligosaccharides (MOS), oligosaccharides of soy, glycosylsucrose (GS), lactosucrose (LS), lactulose (LA), palatinose-oligosaccharides (PAO), malto-oligosaccharides, gums and/or hydrolysates thereof, pectins, starches, and/or hydrolysates thereof.

All probiotic micro-organisms may be used in combination with the non-replicative probiotics that are described in the present invention. Preferably, such an added probiotic may be selected from the group consisting of the genera *Bifidobacterium, Lactobacillus, Lactococcus, Enterococcus, Streptococcus, Propionibacterium, Pediococcus, Escherichia coli, Debaryomyces, Kluyveromyces, Saccharoymces, Schizosaccharomyces, Zygosaccharomyces, Yarrowia, Candida,* in particular selected from the group consisting of the species *Bifidobacterium longum, Bifidobacterium lactis, Bifidobacterium animalis, Bifidobacterium breve, Bifidobacterium infantis, Bifidobacterium bifidum, Bifidobacterium adolescentis, Lactobacillus acidophilus, Lactobacillus casei, Lactobacillus paracasei, Lactobacillus salivarius, Lactobacillus plantarum, Lactobacillus fermentum, Lactobacillus johnsonii, Lactobacillus reuteri, Lactobacillus gasseri, Lactobacillus rhamnosus, Lactococcus ssp. such as Lactococcus lactis, Lactococcus cremoris, Lactococcus diacetylactis, Enterococcus faecium, Enterococcus faecalis, Saccharomyces cerevisiae, Saccharomyces boulardii, Schizosaccharomyces pombe, Kluyveromyces lactis, Yarrowia lypolitica* or mixtures thereof, preferably selected from the group consisting of *Lactobacillus johnsonii* (NCC533; CNCM I-1225), *Bifidobacterium longum* (NCC490; CNCM I-2170), *Bifidobacterium longum* (NCC2705; CNCM I-2618), *Bifidobacterium longum* (NCC3001; ATCC BAA-999), *Bifidobacterium lactis* (NCC2818; CNCM I-3446), *Bifidobacterium breve* (strain A), *Lactobacillus paracasei* (NCC2461; CNCM I-2116), *Lactobacillus rhamnosus* GG (ATCC53103), *Lactobacillus rhamnosus* LPR (NCC4007; CGMCC 1.3724), *Enterococcus faecium* SF 68 (NCIMB10415), and mixtures thereof. All these probiotics may be added in a viable or in a non-replicating form.

The composition prepared by the use of the present invention may be intended for any mammal, but is preferably intended for humans or pets.

For example it may be intended for subjects whose immune system is weakened or transiently depressed such as the neonates, the elderly, subjects submitted to high stress conditions, patients taking immunosuppressive drugs, patients under radiotherapy or chemotherapy.

The composition of the present invention may also be co-administered with vaccines to enhance the effectiveness of the vaccine. Co-administration includes administration by the same or different routes from 3 months before, during, to 2 months after the vaccination regimen.

The compositions of the present invention are administered in an amount sufficient to at least partially treat immune deficiency related disorders, infections and their pathological effects. An amount adequate to accomplish this is defined as "a therapeutically effective dose". Amounts effective for this purpose will depend on a number of factors known to those of skill in the art such as the nature and severity of the disease, the weight and general state of the patient, and the specific probiotic strain used.

In prophylactic applications, compositions according to the invention are administered to a patient susceptible to or otherwise at risk of immune disorders or infections in an amount that is sufficient to at least partially reduce the risk of developing immune disorders. Such an amount is defined to be "a prophylactic effective dose". Again, the precise amounts depend on a number of disease and patient specific factors such as the patient's state of health and weight and the kind of bacterial strain used.

Those skilled in the art will be able to adjust the therapeutically effective dose and/or the prophylactic effective dose appropriately.

In general the composition of the present invention contains non-replicating probiotics in a therapeutically effective dose and/or in a prophylactic effective dose.

Typically, the therapeutically effective dose and/or the prophylactic effective dose is a bacterial mass that corresponds to about 10⁴ to 10¹² cfu per daily dose Consequently, the therapeutically effective and/or the prophylactic effective dose may be in the range of about 0,005 mg - 1000 mg non-replicating probiotics per daily dose.

In terms of numerical amounts, the non-replicating probiotics may be present in the composition in an amount corresponding to between 10² and 10¹² cfu/g of the dry composition. Obviously, non-replicating bacteria do not form colonies; consequently this term is to be understood as the amount of non replicating bacteria that is obtained from 10² and 10¹² cfu/g replicating bacteria. This includes bacteria that are inactivated or dead or present as fragments such as DNA, cytoplasmic content or cell wall materials. In other words, the quantity of bacteria which the composition contains is expressed in terms of the colony forming ability of that quantity of bacteria as if all the bacteria were alive irrespective of whether they are, in fact, non replicating, such as inactivated or dead, fragmented or a mixture of any or all of these states.

Preferably the probiotic is present in an amount equivalent to between 10⁴ to 10¹⁰ cfu/g of dry composition, even more preferably in an amount equivalent to between 10⁵ and 10⁹ cfu/g of dry composition.

The composition may also contain all vitamins and minerals understood to be essential in the daily diet and in nutritionally significant amounts. Minimum requirements have been established for certain vitamins and minerals. Examples of minerals, vitamins and other nutrients optionally present in the composition include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended consumer.

The composition of the present invention may contain at least one protein source, at least one carbohydrate source and at least one lipid source.

Any suitable dietary protein may be used, for example animal proteins (such as milk proteins, meat proteins and egg proteins); vegetable proteins (such as soy proteins, wheat proteins, rice proteins, and pea proteins); partial or total hydrolysates of these proteins, mixtures of free amino acids; or combinations thereof. If hydrolysed proteins are required, the hydrolysis process may be carried out as desired and as is known in the art. Milk proteins such as casein and whey, and soy proteins are particularly preferred. As far as whey proteins are concerned, the protein source may be based on acid whey or sweet whey or mixtures thereof and may include alpha- lactalbumin and beta-lactoglobulin in whatever proportions are desired. Preferably however, in particular if the composition is an infant feeding formula, the protein source is based on modified sweet whey.

If the composition of the present invention contains a protein source, then the amount of protein or protein equivalent in the composition is typically in the range of 1.6-7.5 g/100kcal of the composition.

In particular for nutritional formulas, the protein source should provide that the minimum requirements for essential amino acid content are met.

If the composition contains a carbohydrate source, the kind of carbohydrate to be used is not particularly limited. Any suitable carbohydrate may be used, for example sucrose, lactose, glucose, fructose, corn syrup solids, maltodextrins, starch and mixtures thereof. Combinations of different carbohydrate sources may be used. The carbohydrates may preferably provide 30% to 80% of the energy of the composition. For example, the composition may comprise a carbohydrate source in an amount of 9-18 g/100kcal of the composition.

If the composition contains a lipid source, the kind of lipid to be used is not particularly limited. If the composition includes a lipid source, the lipid source may provide 5% to 70% of the energy of the composition. Long chain n-3 and/or n-6 polyunsaturated fatty acids, such as DHA, ARA and/or EPA may be added. A suitable fat profile may be obtained using a blend of canola oil, corn oil, high-oleic acid sunflower oil and medium chain triglyceride oil. The composition may comprise a lipid source in an amount of 1.5-7 g/100kcal of the composition.

Dietary fibre may be added as well. They may be soluble or insoluble and in general a blend of the two types is preferred. Suitable sources of dietary fibre include soy, pea, oat, pectin, guar gum, arabic gum, fructooligosaccharides, galacto-oligosaccharides, sialyl-lactose and oligosaccharides derived from animal milks. A preferred fibre blend is a mixture of inulin with shorter chain fructo-oligosaccharides.

Food products according to the present invention include drinks, in particular milk- or yoghurt based drinks; dairy products, such as yoghurts, ice creams, or cheese based products; fruit juices and soft drinks; nutritional formulas, such as those for complete nutrition, for example for infants, children, teenagers, adults, the elderly or the critically ill subjects; cereals and cereal bars; milk based powders, powders to be dissolved in a water- of milk-based liquid, coffee creamers, soups, spreadings, for example.

A composition of the present invention comprises non-replicating probiotics.

The non-replicating probiotics may be selected from the group of genera consisting of lactobacilli, bifidobacteria or combinations thereof, such as the species *Lactobacillus paracasei, Lactobacillus rhamnosus, Bifidobacterium longum, Bifidobacterium lactis, Bifidobacterium breve* or combinations thereof, for example the strains *Lactobacillus paracasei* NCC2461, *Lactobacillus rhamnosus* NCC4007, *Bifidobacterium longum* NCC3001, *Bifidobacterium lactis* NCC2818, *Bifidobacterium breve* strain A or combinations thereof.

The probiotics may be rendered non-replicating by a heat treatment, for example, in the temperature range of about 70-150 °C for about 3 minutes - 2 hours, preferably in the range of 80-140°C from 5 minutes - 40 minutes.

Such a composition may be prepared by any manner known in the art. For example, if the composition is a nutritional formula, such as an infant feeding formula it may be prepared by blending together a protein source, a carbohydrate source, and a fat source in appropriate proportions. If used, emulsifiers may be included in the blend. Vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.

The liquid mixture may then be thermally treated to reduce bacterial loads. For example, the liquid mixture may be rapidly heated to a temperature in the range of about 70-150 °C for about 3 minutes - 2 hours, preferably in the range of 80-140°C from 5 minutes - 40 minutes. This may be carried out by steam injection or by heat exchanger; for example a plate heat exchanger.

The liquid mixture may then be cooled to about 60°C to 85°C; for example by flash cooling. The homogenised mixture may then be further cooled to add any heat sensitive components; such as vitamins and minerals.

The pH and solid content of the homogenised mixture is conveniently standardised at this point. The homogenised mixture is transferred to a suitable drying apparatus such as a spray drier, freeze drier, or roller drier and converted to powder. The powder should have a moisture content of less than about 5% by weight.

The probiotics may be cultured according to any suitable method known to those skilled in the art and prepared for addition to the nutritional composition by freeze-drying or spray-drying for example. The probiotics may then be added to the composition before the composition is heat treated to reduce bacterial loads. This will automatically render the probiotics non-replicative.

In general, using non-replicating probiotics has the advantage that the bacteria may conveniently be added to any composition before a heat treatment step, for example a sterilization step. Such sterilization may be achieved by mild heat treatment for a longer time. This way, it can be avoided that bacterial compositions have to be added to a product after a heating step, so that - at the same time - one production step is saved and the risk of bacterial contamination after the heating step is reduced.

Alternatively, of course, the probiotics may also be heat treated individually and then added to the composition as non-replicating probiotics. Addition of non-replicating probiotics delivering a health benefit will also allow hot reconstitution of powdered food compositions.

If any probiotics are to be added in addition to the composition, the selected probiotic(s) may be cultured according to any suitable method and prepared for addition to the composition by freeze-drying or spray-drying for example. Alternatively, bacterial preparations can be bought from specialist suppliers already prepared in a suitable form for addition to food products.

The inventors describe here for the first time a method to increase the effectiveness of probiotics to treat or prevent immune deficiency related disorders. The method involves rendering at least a part of the probiotics non-replicating by heat treatment.

One embodiment of the present invention is therefore a method to increase the effectiveness of probiotics, for example selected from the group consisting of the genera lactobacilli, bifidobacteria or combinations thereof, such as the species *Lactobacillus paracasei, Lactobacillus rhamnosus, Bifidobacterium longum, Bifidobacterium lactis, Bifidobacterium breve* or combinations thereof, for example the strains *Lactobacillus paracasei* NCC2461, *Lactobacillus rhamnosus* NCC4007, *Bifidobacterium longum* NCC3001, *Bifidobacterium lactis* NCC2818, *Bifidobacterium breve* strain A or combinations thereof, to treat or prevent disorders related to a compromised immune defence after administration comprising the step of subjecting the probiotics to a treatment that renders at least a part of the probiotics, for example bifidobacteria or lactobacilli non-replicating.

This treatment may be a heat treatment, for example in the temperature range of about 70-150 °C for about 3 minutes - 2 hours, preferably in the range of 80-140°C from 5 minutes - 40 minutes, prior to administration.

The treatment, for example the heat treatment of the probiotics, may result in rendering at least 95 %, preferably at least 97,5 %, more preferably at least 99 %, most preferably all of the probiotics non-replicating.

The method of the present invention may be used to increase the effectiveness of probiotics to treat or prevent a disorders related to a compromised immune defence.

For an improved stability of the bacterial preparation as well as for an improved taste, the non-replicating probiotics may be added to a composition intended for consumption prior to ingestion of the composition. This way a composition comprising the non-replicating probiotics can be ingested and the further constituents of the composition can be used to adapt the composition to its intended purpose.

Alternatively, the method may involve the step of adding viable probiotics to a composition and then subjecting the probiotic containing composition to a treatment that renders at least a part of the probiotics non-replicating prior to administration.

Those skilled in the art will understand that they can freely combine all features of the present invention described herein, without departing from the scope of the invention as disclosed. In particular, features described for the use of the present invention may be applied to the composition and to the method of the present invention and vice versa.

Further advantages and features of the present invention are apparent from the following Examples and Figures.

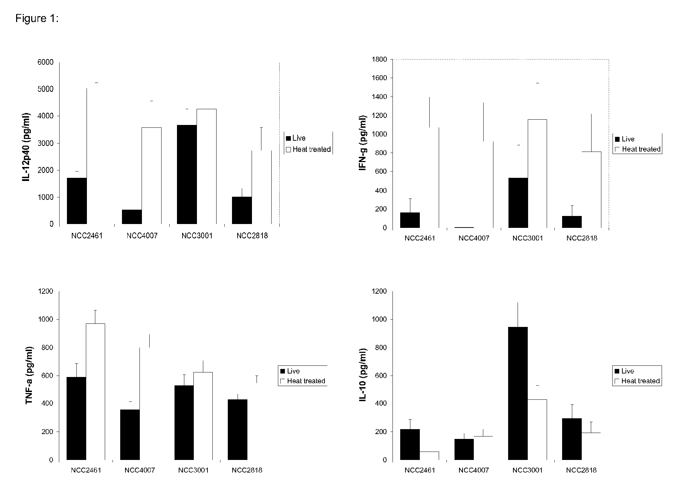

Figure 1 shows the enhancement of *in vitro* cytokine secretion from human PBMCs stimulated with heat treated bacteria.

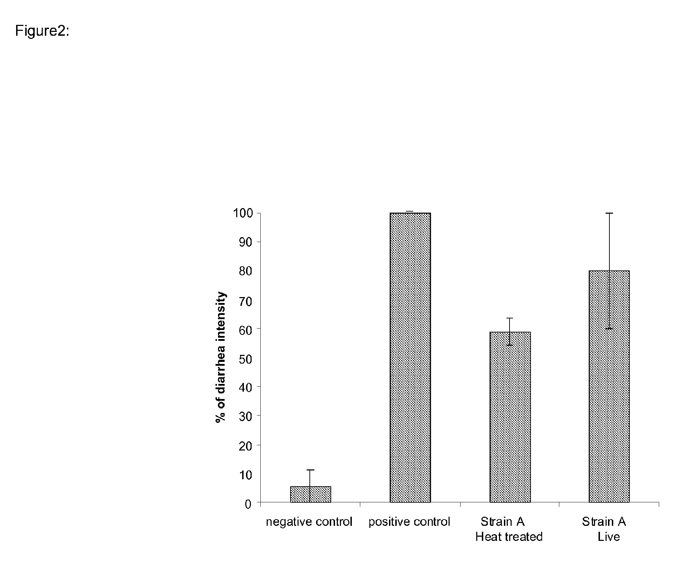

Figure 2 shows the percentage of diarrhea intensity observed in OVA-sensitized mice challenged with saline (negative control), OVA-sensitized mice challenged with OVA (positive control) and OVA-sensitized mice challenged with OVA and treated with heat-treated or live *Bifidobacterium breve* strain A. Results are displayed as the percentage of diarrhea intensity (Mean ± SEM calculated from 4 independent experiments) with 100 % of diarrhea intensity corresponding to the symptoms developed in the positive control (sensitized and challenged by the allergen) group.

### Examples:

### Methodology

### Bacterial preparations:

Five probiotic strains were used to investigate the immune boosting properties of non-replicating probiotics: 3 bifidobacteria (*B. longum* NCC3001, *B. lactis* NCC2818, *B. breve* strain A) and 2 lactobacilli (*L. paracasei* NCC2461, *L. rhamnosus* NCC4007).

Bacterial cells were grown on MRS in batch fermentation at 37°C for 16-18h without pH control. Bacterial cells were spun down (5,000 x g, 4°C) and resuspended in phosphate buffer saline prior to be diluted in saline water in order to reach a final concentration of around 10E10 cfu/ml. *B. longum* NCC3001, *B. lactis* NCC2818, *L. paracasei* NCC2461, *L. rhamnosus* NCC4007 were heat treated at 85°C for 20 min in a water bath. *B. breve* strain A was heat treated at 90°C for 30 minutes in a water bath. Heat treated bacterial suspensions were aliquoted and kept frozen at -80°C until use. Live bacteria were stored at -80°C in PBS-glycerol 15% until use.

### In vitro immunoprofiling of bacterial preparations

The immune profiles of live and heat treated bacterial preparations (i.e. the capacity to induce secretion of specific cytokines from human blood cells *in vitro*) were assessed. Human peripheral blood mononuclear cells (PBMCs) were isolated from blood filters. After separation by cell density gradient, mononuclear cells were collected and washed twice with Hank's balanced salt solution. Cells were then resuspended in lscove's Modified Dulbecco's Medium (IMDM, Sigma) supplemented with 10% foetal calf serum (Bioconcept, Paris, france), 1% L-glutamine (Sigma), 1% penicillin/streptomycin (Sigma) and 0.1% gentamycin (Sigma). PBMCs (7x10⁵ cells/well) were then incubated with live and heat treated bacteria (equivalent 7x10⁶ cfu/well) in 48 well plates for 36h. The effects of live and heat treated bacteria were tested on PBMCs from 8 individual donors splitted into two separate experiments.

After 36h incubation, culture plates were frozen and kept at -20°C until cytokine measurement. Cytokine profiling was performed in parallel (i.e. in the same experiment on the same batch of PBMCs) for live bacteria and their heat-treated counterparts.

Levels of cytokines (IFN-γ, IL-12p40, TNF-α and IL-10) in cell culture supernatants after 36h incubation were determined by ELISA (R&D DuoSet Human IL-10, BD OptEIA Human IL12p40, BD OptEIA Human TNF, BD OptEIA Human IFN-y) following manufacturer's instructions. IFN-γ, IL-12p40 and TNF-α are pro-inflammatory cytokines, whereas IL-10 is a potent anti-inflammatory mediator. Results are expressed as means (pg/ml) +/- SEM of 4 individual donors and are representative of two individual experiments performed with 4 donors each.

*In vivo effect of live and heat treated Bifidobacterium breve* strain A *in prevention of allergic diarrhea*

A mouse model of allergic diarrhea was used to test the Th1 promoting effect of *B. breve* strain A (Brandt E.B et al. JCI 2003; 112(11): 1666-1667). Following sensitization (2 intraperitoneal injections of Ovalbumin (OVA) and aluminium potassium sulphate at an interval of 14 days; days 0 and 14) male Balb/c mice were orally challenged with OVA for 6 times (days 27, 29, 32, 34, 36, 39) resulting in transient clinical symptoms (diarrhea) and changes of immune parameters (plasma concentration of total IgE, OVA specific IgE, mouse mast cell protease 1, i.e MMCP-1). *Bifidobacterium breve* strain A live or heat treated at 90°C for 30min, was administered by gavage 4 days prior to OVA sensitization (days -3, -2, -1, 0 and days 11, 12, 13 and 14) and during the challenge period (days 23 to 39). A daily bacterial dose of around 10⁹ colony forming units (cfu) or equivalent cfu/mouse was used.

### Results

### Induction of secretion of 'pro-inflammatory' cytokines after heat treatment

The ability of heat treated bacterial strains to stimulate cytokine secretion by human peripheral blood mononuclear cells (PBMCs) was assessed *in vitro.* The immune profiles based on four cytokines upon stimulation of PBMCs by heat treated bacteria were compared to that induced by live bacterial cells in the same *in vitro* assay.

The heat treated preparations were plated and assessed for the absence of any viable counts. Heat treated bacterial preparations did not produce colonies after plating.

Live probiotics induced different and strain dependent levels of cytokine production when incubated with human PBMCs (Figure 1). Heat treatment of probiotics modified the levels of cytokines produced by PBMCs as compared to their live counterparts. Heat treated bacteria induced more pro-inflammatory cytokines (TNF-α, IFN-γ, IL-12p40) than their live counterparts do. By contrast heat treated bacteria induced similar or lower amounts of IL-10 compared to live cells (Figure 1). These data show that heat treated bacteria are more able to stimulate the immune system than their live counterparts and therefore are more able to boost weakened immune defences. In other words the *in vitro* data illustrate an enhanced immune boost effect of bacterial strains after heat treatment.

In order to illustrate the enhanced effect of heat-treated *B. breve* strain A (compared to live cells) on the immune system, both live and heat treated *B. breve* strain A were tested in an animal model of allergic diarrhea.

As compared to the positive control group, the intensity of diarrhea was significantly and consistently decreased after treatment with heat treated *B. breve* strain A (41.1 % ± 4.8) whereas the intensity of diarrhea was lowered by only 20 ± 28.3 % after treatment with live *B. breve* strain A. These results demonstrate that *heat-treated B.* breve strain A exhibits an enhanced protective effect against allergic diarrhea than its live counterpart (Figure 2).

As a consequence, the ability of probiotics to enhance the immune defences was shown to be improved after heat treatment.

## Claims

1. Use of probiotics that are rendered non-replicating by a heat treatment of at least about 70°C for at least about 3 minutes for the preparation of a composition to treat or prevent disorders related to a compromised immune defence.

2. Use in accordance with claim 1, wherein the probiotics are selected from the group consisting of the genera lactobacilli, bifidobacteria or combinations thereof, such as the species *Lactobacillus paracasei, Lactobacillus rhamnosus, Bifidobacterium longum, Bifidobacterium lactis, Bifidobacterium breve* or combinations thereof, for example the strains *Lactobacillus paracasei* NCC2461, *Lactobacillus rhamnosus* NCC4007, *Bifidobacterium longum* NCC3001, *Bifidobacterium lactis* NCC2818, *Bifidobacterium breve* strain A or combinations thereof.

3. Use in accordance with one of the preceding claims, wherein the heat treatment is carried out in the temperature range of about 70-150 °C for about 3 minutes - 2 hours, preferably in the range of 80-140°C from 5 minutes - 40 minutes.

4. Use in accordance with one of the preceding claims, wherein the disorder is selected from the group consisting of infections, in particular bacterial, viral, fungal and/or parasite infections; phagocyte deficiencies; low to severe immunodepression levels such as those induced by stress or immunodepressive drugs, chemotherapy or radiotherapy; natural states of less immunocompetent immune systems such as those of the neonates or elderly; allergies; and combinations thereof.

5. Use in accordance with one of the preceding claims wherein the composition is selected from the group consisting of food compositions, food products including pet foods, drinks, nutritional formulas, feeding formulas, nutraceuticals, food additives, pharmaceutical compositions, cosmetical compositions, medicaments.

6. Use in accordance with one of the preceding claims wherein the composition further comprises a pain or fever relieving agent, a stabilizing agent, a flavouring agent, a colouring agent, a lubricant, a probiotic and/or a prebiotic.

7. Use in accordance with one of the preceding claims wherein the composition is intended for humans or pets.

8. Use in accordance with one of the preceding claims wherein the composition contains an amount of non-replicating probiotics corresponding to about 10⁴ to 10¹² cfu per daily dose.

9. Use in accordance with one of the preceding claims wherein the composition contains about 0,005 mg - 1000 mg non-replicating probiotics per daily dose.

10. Composition comprising non-replicating probiotics, wherein probiotics are selected from the group consisting of the genera lactobacilli, bifidobacteria or combinations thereof, such as the species *Lactobacillus paracasei, Lactobacillus rhamnosus, Bifidobacterium longum, Bifidobacterium lactis, Bifidobacterium breve* or combinations thereof, for example the strains *Lactobacillus paracasei* NCC2461, *Lactobacillus rhamnosus* NCC4007, *Bifidobacterium longum* NCC3001, *Bifidobacterium lactis* NCC2818, *Bifidobacterium breve* strain A or combinations thereof, wherein the probiotics are rendered non-replicating by a heat treatment in the temperature range of about 70-150 °C for about 3 minutes - 2 hours, preferably in the range of 80-140°C from 5 minutes to 40 minutes.

11. Composition in accordance with claim 10 containing about 0,005 mg - 1000 mg non-replicating probiotics per daily dose.

12. Method to increase the effectiveness of probiotics, in particular probiotics selected from the group consisting of the genera lactobacilli, bifidobacteria or combinations thereof, such as the species *Lactobacillus paracasei, Lactobacillus rhamnosus, Bifidobacterium longum, Bifidobacterium lactis, Bifidobacterium breve* or combinations thereof, for example the strains *Lactobacillus paracasei* NCC2461, *Lactobacillus rhamnosus* NCC4007, *Bifidobacterium longum* NCC3001, *Bifidobacterium lactis* NCC2818, *Bifidobacterium breve* strain A or combinations thereof, to treat or prevent disorders related to a compromised immune defence comprising the step of subjecting the probiotics to a heat treatment, preferably in the temperature range of about 70-150 °C for about 3 minutes - 2 hours, preferably in the range of 80-140°C from 5 minutes - 40 minutes prior to administration, wherein the heat treatment of the probiotics results in rendering at least 95 %, preferably at least 97,5 %, more preferably at least 99 %, most preferably all of the probiotics non-replicating.

13. Method in accordance with claim 12 to increase the effectiveness of probiotics to treat or prevent disorders related to a compromised immune defence.

14. Method in accordance with one of claims 12-13, comprising the step of adding non-replicating probiotics to a composition intended for administration.

15. Method in accordance with one of claims 12-13, and/or comprising the step of adding viable probiotics to a composition and subjecting the probiotics containing composition to a heat treatment prior to administration.
